# EUROPÄISCHE PATENTSCHRIFT

(11) **EP 3 542 721 B1**
(45) Veröffentlichungstag und Bekanntmachung des Hinweises auf die Patenterteilung: **19.02.2025**
(21) Anmeldenummer: 18163687.9
(22) Anmeldetag: 23.03.2018
(51) Int. Cl.: A61B 6/00, A61B 6/03, G06N 3/08, G06T 11/00, A61B 6/50, G06N 3/045

(54) **VERFAHREN ZUR BEARBEITUNG VON PARAMETERN EINES MASCHINENLERNVERFAHRENS SOWIE REKONSTRUKTIONSVERFAHREN**
METHOD FOR PROCESSING PARAMETERS OF A MACHINE LEARNING METHOD AND RECONSTRUCTION METHOD
PROCÉDÉ DE TRAITEMENT DES PARAMÈTRES D'UN PROCÉDÉ D'APPRENTISSAGE PAR MACHINE AINSI QUE PROCÉDÉ DE RECONSTRUCTION

(43) Veröffentlichungstag der Anmeldung: 25.09.2019
(73) Patentinhaber: Siemens Healthineers AG, 91301 Forchheim (DE)
(72) Erfinder: Fournié, Eric, 91054 Erlangen (DE)
(74) Vertreter: Siemens Healthineers Patent Attorneys

(56) Entgegenhaltungen:
- WO-A1-2017/223560
- US-A1- 2016 000 383
- WÜRFL TOBIAS ET AL: "Deep Learning Computed Tomography", 2 October 2016, MEDICAL IMAGE COMPUTING AND COMPUTER-ASSISTED INTERVENTION - MICCAI 2015 : 18TH INTERNATIONAL CONFERENCE, MUNICH, GERMANY, OCTOBER 5-9, 2015; PROCEEDINGS; [LECTURE NOTES IN COMPUTER SCIENCE; LECT.NOTES COMPUTER], SPRINGER INTERNATIONAL PUBLISHING, CH, ISBN: 978-3-540-70543-7, ISSN: 0302-9743, XP047364505

## Beschreibung

Die Erfindung betrifft ein Verfahren zur Bearbeitung von Parametern eines Maschinenlernverfahrens zur Bereitstellung eines Korrekturdatensatzes für eine Bewegungskorrektur eines CT-Bilddatensatzes eines bei dessen Aufnahme bewegten Objekts. Ferner betrifft die Erfindung ein Rekonstruktionsverfahren, welches das bearbeitete Maschinenlernverfahrens verwendet sowie eine Vorrichtung.

Bei der Bildgebung mittels eines Computertomographiegeräts (CT-Scanner) verfahren eine Röntgenquelle und/oder ein Detektor auf einer vordefinierten Bahn, insbesondere auf einer Kreis- oder Helixbahn, relativ zu einem zu untersuchenden Objekt. Dabei wird ein (CT-)Bilddatensatz aus unterschiedlichen (Aufnahme-) Winkeln aufgenommen. Insbesondere im Bereich der medizinischen Diagnostik, in welcher ein Organ eines Patienten wie beispielsweise das Herz untersucht wird, ist dabei eine Aufnahmezeit des CT-Bilddatensatzes für den gesamten aufzunehmenden Winkelbereich vergleichsweise groß, so dass sich das Organ oder zumindest ein Teil des Organs während der Aufnahme des CT-Bilddatensatzes bewegt.

In Folge dessen zeigen sich Bewegungsartefakte des bewegten Objekts in einem aus dem CT-Bilddatensatz rekonstruierten (Rekonstruktions-)Bilddatensatz. Nachteilig ist aufgrund solcher Bewegungsartefakte eine zuverlässige Diagnose erschwert. So stellen die Bewegungsartefakte eine Fehlerquelle bei einer Diagnose dar. Beispielsweise ist die Struktur von Herzarterien aufgrund der Bewegung des Herzens während der Aufnahme des CT-Bilddatensatzes in einem als Bilddarstellung (Bild) dargestellten Rekonstruktionsbilddatensatz nicht klar erkennbar, insbesondere unscharf, verzerrt und/oder verformt dargestellt.

Es sind hardwarebezogene Lösungen wie beispielsweise die Verwendung eines zweiten Detektors und einer zweiten Röntgenquelle oder einer Erhöhung der Drehgeschwindigkeit eines die Röntgenquelle und den Detektor tragenden Drehkranzes (Gantry) bekannt. Diese Lösungen sind allerdings vergleichsweise teuer.

Es sind ferner Verfahren bekannt, welche einen Korrekturdatensatz für den aufgenommenen CT-Bilddatensatz bestimmten, um die Bewegungsartefakte zu reduzieren. So ist beispielsweise aus "Motion compensation in the region of the coronary arteries based on partial angle reconstruction from short scan data" von J. Hahn et al. in "Medical Physics", 2017 Nov., Vol. 44(11), S. 5795-5813 ein Verfahren zur Reduzierung von Bewegungsartefakten einer Rekonstruktion von Herzkranzgefäßen bekannt. Hierbei wird die Bewegung entlang einer Mittellinie des entsprechenden Herzkranzgefäßes mittels eines Bewegungsvektorfeldes modelliert, dessen Parameter mittels einer Kostenfunktion abgeschätzt wird.

Beispielsweise ist in "Nonrigid registration-based coronary artery motion correction for cardiac computed tomography" von R. Bhagalia et al. in "Medical Physics", 2012 Juli, Vol. 39(7), S. 4245-4254 ferner offenbart, dass eine Bewegungskompensation von Herzkranzgefäßen mittels einer dreidimensionalen Deformation ("warping") einer Serie von partiellen Rekonstruktionen anhand von geschätzten Bewegungsvektorfeldern realisiert wird.

Weiterhin ist aus "Cardiac motion corrected iterative conebeam CT reconstruction using a semi-automatic minimum cost path-based coronary centerline extraction" von A. A. Isola et. al. in "Computerized medical imaging and graphics", 2012 April, Vol. 36(3), S. 215-226, ein Verfahren bekannt, dass zur Erzeugung von Bewegungsartefakt freier Rekonstruktionen von Herzkranzgefäßen Bewegungsvektorfelder nutzt, welche aus einer Anzahl an Mittellinien der entsprechenden Herzkranzgefäße bestimmt werden.

Diesen Verfahren ist gemeinsam, dass mittels eines Algorithmus ein Bewegungsvektorfeld (motion vector field) als Korrekturdatensatz für die Bewegung des Objekts bei der Aufnahme des CT-Bilddatensatzes bestimm wird. Die Verfahren sind jedoch, insbesondere aufgrund der Bestimmung des Korrekturdatensatzes mittels des jeweiligen Algorithmus, vergleichsweise zeitaufwändig, so dass zwischen der Aufnahme des CT-Bilddatensatzes und der Bereitstellung des mittels des Korrekturdatensatzes bewegungsreduzierten Rekonstruktionsbilddatensatzes für eine Diagnose vergleichsweise viel Zeit vergeht.

Aus der Druckschrift DE 10 2009 007 236 A1 ist ein Verfahren zur Abtastung eines sich bewegenden Untersuchungsobjektes mit einem CT-System bekannt, bei welchem während einer rotierenden Bewegung eines Sender-/Empfängerpaares um das Untersuchungsobjekt Daten erfasst werden. Ferner werden aus den Daten mittels eines iterativen Algorithmus Schnittbilder des Untersuchungsobjektes ermittelt, wobei bei dem iterativen Algorithmus Bewegungsinformationen betreffend die Bewegung des Untersuchungsobjektes während der Datenerfassung berücksichtigt werden.

Aus der Druckschrift DE 10 2011 007 529 A1 ist ein Verfahren, ein Strahlentherapiesystem und eine Kombination aus CT-System und Strahlentherapiesystem zur Bestimmung eines Bewegungsprofils eines sich in einem Untersuchungsobjekt bewegenden Objektes mit einem relativ zum Untersuchungsobjekt verschiebbarem Strahler-Detektor-System bekannt, wobei die folgenden Verfahrensschritte ausgeführt werden:
- Abtastung des Untersuchungsobjektes im Bereich des sich bewegenden Objektes während einer Relativverschiebung des Strahler-Detektor-Systems zum Untersuchungsobjekt und erzeugen eines Pixeldatensatzes mit Schwächungswerten über die Zeit,
- Entfernung ortsfester Strukturen aus dem Pixeldatensatz,
- Bestimmung eines durch das bewegte Objekt induzierten Schwächungswertes in jeder Detektorzeile zu einer Vielzahl von aufeinander folgenden Zeitpunkten der Abtastung und Bildung eines 3D-Datensatzes aus den Werten des Schwächungsmaximums der Detektorzeilen über die Detektorzeilen und die Auslesezeitpunkte der Abtastung, und
- Bestimmung mindestens eines der Werte aus nachfolgender Liste aus dem Ergebnisdatensatz: Frequenz und/ oder Phase und/oder Amplitude der Bewegung des Objektes, Aufenthaltsbereich des Objektes während der Abtastung, Position des Objektes an einer vorgegebenen Phase der Bewegung.

Aus der Druckschrift DE 10 2011 083 647 A1 ist ein Verfahren zur Erzeugung eines bewegungskompensierten CT-Bilddatensatzes bekannt, wobei:
- ein Projektionsdatensatzes eines CT-Systems aus einer vorgegebenen Bewegungsphase und einem Projektionswinkelbereich, welcher die unmittelbare Rekonstruktion eines CT-Bilddatensatzes erlaubt, erfasst wird,
- iterativ das Bewegungsfeld bestimmt wird, durch:
- mehrfache Rekonstruktion des einen CT-Bilddatensatzes mit einer ersten Bildauflösung mit einem bewegungskompensierenden Rekonstruktionsverfahren unter Verwendung eines ersten analytischen Rekonstruktionsalgorithmus und unterschiedlicher Bewegungsfelder aus jeweils einer Vielzahl ortsspezifischer Bewegungsvektoren,
- und Ermittlung des Bewegungsfeldes unter Verwendung mindestens einer vorgegebenen Zwangsbedingungen,
- und Rekonstruktion eines endgültigen CT-Bilddatensatzes mit einer zweiten Bildauflösung unter Verwendung eines bewegungskompensierenden Rekonstruktionsverfahrens auf der Basis eines zweiten Rekonstruktionsalgorithmus und des ermittelten Bewegungsfeldes.

Aus der WO 2017/223560 A1 ist ferner bekannt, für eine Bildrekonstruktion ein Maschinenlernverfahren, beispielsweise "deep learning" für ein künstliches neuronales Netz, heranzuziehen.

In "Deep Learning Computed Tomography" von Würfl, T. et al. in "International Conference on Medical Image Computing and Computer-Assisted Intervention", Springer, Cham, 2016 ist offenbart, dass eine Bildrekonstruktion mittels eines neuronalen Netzwerks realisiert werden kann. Insbesondere wird gezeigt, dass das neuronale Netz Filter und Gewichtungen zur Reduktion von Rekonstruktionsfehlern bei CT-Aufnahmen mit einem eingeschränkten Winkelbereich lernt.

In der US 2016/000383 A1 ist ein Verfahren offenbart, bei welchem eine Bewegung während einer medizinischen Bildgebung erfasst wird. Hierbei wird zunächst die Akquisition von Bilddaten initiiert und physiologische Signale eines Patienten erfasst. Anhand dieser Signale wird bestimmt, ob eine Bewegung des Patienten wahrscheinlich ist und gegebenenfalls die Akquisition der Bilddaten geändert.

Der Erfindung liegt die Aufgabe zugrunde, ein Verfahren anzugeben, welches eine vergleichsweise schnelle Bereitstellung eines Korrekturdatensatzes für den entsprechenden CT-Bilddatensatz ermöglicht. Ferner soll ein Rekonstruktionsverfahren zur Erzeugung eines Rekonstruktionsbilddatensatzes angegeben werden. Ferner soll eine Vorrichtung zur Ausführung eines der oder beider Verfahren angegeben werden.

Diese Aufgabe wird bezüglich des Verfahrens erfindungsgemäß gelöst durch die Merkmale des Anspruchs 1. Bezüglich des Rekonstruktionsverfahrens wird die Aufgabe erfindungsgemäß gelöst durch die Merkmale des Anspruchs 5 und bezüglich der Vorrichtung wird die Aufgabe erfindungsgemäß gelöst durch die Merkmale des Anspruchs 6. Vorteilhafte Weiterbildungen und Ausgestaltungen sind Gegenstand der Unteransprüche.

Hierzu werden in einem Verfahren Parameter eines Maschinenlernverfahrens bearbeitet, welches Maschinenlernverfahren der Bereitstellung eines Korrekturdatensatzes für eine Bewegungskorrektur eines CT-Bilddatensatzes eines bei dessen Aufnahme bewegten Objekts dient. Die Bearbeitung der Parameter des Maschinenlernverfahrens wird auch als Training bezeichnet. Die Parameter werden vor Beginn des Trainings, beispielsweise mit Zufallszahlen, initialisiert. Der CT-Bilddatensatz repräsentiert einen Intensitätsverlauf der auf einen Detektor einfallenden Röntgenstrahlung aus unterschiedlichen (Aufnahme-) Winkeln. Der CT-Bilddatensatz ist insbesondere ein so genanntes Sinogramm.

Verfahrensgemäß wird zunächst ein Trainingsdatensatz mit einer Anzahl an Referenzbilddatensätzen zum Einleiten (Einspeisen) in das Maschinenlernverfahren bereitgestellt und eingeleitet. Mit anderen Worten wird der Trainingsdatensatz als Eingangsdatensatz (Input) an das Maschinenlernverfahren gegeben.

Ein Referenzbilddatensatz ist ein vorbekannter CT-Bilddatensatz, welcher für die Bearbeitung der Parameter des Maschinenlernverfahrens bereitgestellt ist. Beispielsweise werden klinische Aufnahmen eines Objekts wie eines Herzes oder aus Aufnahmen eines sog. Phantoms hierzu herangezogen. Alternativ werden solche Referenzbilddatensätze beispielsweise durch eine Berechnung oder durch eine Simulation eines bewegten Objekts erzeugt.

Verfahrensgemäß wird des Weiteren eine Iteration mit mindestens einem Iterationsschritt durchgeführt. In jedem der Iterationsschritte der Iteration wird ein Korrekturdatensatz für jeden der Referenzbilddatensätze mittels des Maschinenlernverfahrens bestimmt und ein Ergebnis einer Kostenfunktion ermittelt. Die Kostenfunktion ist dabei abhängig von den in diesem Iterationsschritt bestimmten Korrekturdatensätzen.

Ein Korrekturdatensatz ist insbesondere als ein Bewegungsvektorfeld ausgebildet, welches eine Bewegung des Objektes in einem Bilddatensatz repräsentiert.

Die Kostenfunktion ist eine mathematische Funktion, welche eine Abweichung (Unterschied) zwischen mittels des Maschinenlernverfahrens ermittelter Größen oder Daten (der Prognose) zu entsprechenden Sollwerten oder Istwerten repräsentiert. Mit anderen Worten quantifiziert die Kostenfunktion einen Fehler der Prognose des Maschinenlernverfahrens. Beispielsweise ist die Kostenfunktion die mittlere quadratische Abweichung zwischen den ermittelten Größen und den entsprechenden Sollwerten.

In Abhängigkeit des Ergebnisses der Kostenfunktion wird die Iteration beendet oder ein Parameter des Maschinenlernverfahrens geändert. Insbesondere wird hierzu ein Schwellenwert vorgegeben. Der Schwellenwert repräsentiert also einen maximal zugelassenen Fehler der mittels des Maschinenlernverfahrens gemachten Prognose. Bei Unterschreiten des Schwellenwertes durch das Ergebnis der Kostenfunktion wird die Iteration für den jeweiligen Parameter beendet, andernfalls ein weiterer Iterationsschritt mit geändertem Parameter durchgeführt. Nach dem Beenden der Iteration wird die Iteration für den nächsten Referenzbilddatensatz durchgeführt.

Besonders bevorzugt wird der Parameter so geändert, dass das Ergebnis der Kostenfunktion verringert wird. Insbesondere erfolgt die Änderung der Parameter nach der sog. "stochastic gradient descent"- Methode. Folglich ist ein Fehler einer Prognose, welche mittels des Maschinenlernverfahrens mit geändertem Parameter bestimmten wird, verringert.

Beispielsweise kann der Trainingsdatensatz in Teil-Trainingsdatensätze unterteilt werden bzw. sein. Dann wird für jeden der Teil-Trainingsdatensätze jeweils eine Iteration durchgeführt, wobei in jedem Iterationsschritt der jeweiligen Iteration in analoger Weise in Abhängigkeit des Ergebnisses der Kostenfunktion die Parameter bearbeitet werden oder die Iteration des jeweiligen Teil-Trainingsdatensatzes beendet wird. Dies ist insbesondere dann vorteilhaft, wenn eine das Maschinenlernverfahren ausführende Vorrichtung einen lediglich vergleichsweise kleinen Speicher aufweist, so dass nicht alle Referenzbilddatensätze in einer einzigen Iteration berücksichtigt werden können oder dies zu einer vergleichsweise langen Bearbeitungsdauer führen würde.

Das Maschinenlernverfahren, auch als maschinelles Lernen bezeichnet, ist hierbei beispielsweise als ein Lern- oder Prognosealgorithmus ausgeführt, welcher mittels eines Controllers ausgeführt wird. Vorteilhaft erkennt dieses Maschinenlernverfahren nach Beenden einer Trainingsphase mit vorbekannten Trainingsdatensätzen Muster und Gesetzmäßigkeiten. Es werden also die vorbekannten Referenzbilddatensätze des eingespeisten Trainingsdatensatzes und hierzu bestimmte Korrekturdatensätze nicht auswendig gelernt bzw. hinterlegt oder abgespeichert, sondern Verallgemeinerungen und/oder Zusammenhänge bestimmt. So kann mittels des insbesondere als Prognosealgorithmus ausgebildeten Maschinenlernverfahrens ein unbekannter Datensatz beurteilt bzw. ausgewertet werden. Nach Beenden der Trainingsphase ist es somit ermöglicht, mittels des trainierten Maschinenlernverfahrens vergleichsweise schnell einen eingespeisten Datensatz auszuwerten und entsprechende Ergebnisse dieser Auswertung, hier also einen Korrekturdatensatz für eine Bewegungskorrektur eines eingespeisten CT-Bilddatensatzes, auszugeben. Bei einer Verwendung eines solchen Maschinenlernverfahrens für die Bereitstellung eines Korrekturdatensatzes als Ergebnis der Auswertung eines diesem zugeführten CT-Bilddatensatzes erfolgt somit vorteilhaft in vergleichsweise kurzer Zeit. Folglich können CT-Bilddatensätze vergleichsweise schnell korrigiert und für eine Diagnose bereitgestellt werden.

Erfindungsgemäß umfasst das Verfahren in einer ersten Variante des Verfahrens als Alternative zur im Folgenden beschriebenen zweiten Variante, dass zur Bestimmung des Ergebnisses der Kostenfunktion Differenzen aus den bestimmten Korrekturdatensätzen und vorbekannten Trainings-Korrekturdatensätzen herangezogen werden. Die Trainings-Korrekturdatensätze sind dabei im Trainingsdatensatz hinterlegt und dem entsprechenden Referenzbilddatensatz zugeordneten. Insbesondere wird als das Ergebnis der Kostenfunktion ein Mittelwert der quadratischen Differenzen herangezogen, alternativ jedoch werden die Differenzen als Argumente einer mathematischen Funktion verwendet. In dieser ersten Variante weist der Trainingsdatensatz also vorbekannte Trainings-Korrekturdatensätze, deren jeder dem entsprechenden Referenzdatensatz zugeordnet ist. Diese Trainings-Korrekturdatensätze sind beispielsweise mittels eines der eingangs als Stand der Technik genannten Verfahren aus dem entsprechenden Referenzdatensatz ermittelt worden, oder alternativ mittels einer Simulation des Referenzdatensatzes und des zugeordneten Trainings-Korrekturdatensatz bestimmt worden.

Alternativ umfasst das Verfahren erfindungsgemäß in einer zweiten Variante des Verfahrens, dass zur Ermittlung des Ergebnisses der Kostenfunktion zunächst jeder der Referenzbilddatensätze entsprechend des zugeordneten und bestimmten Korrekturdatensatzes deformiert (angepasst) wird, insbesondere wird der Intensitätsverlauf (Intensitätsverteilung) des Referenzbilddatensatz entsprechend des bestimmten Korrekturdatensatzes geändert. Beispielsweise werden hierzu aus dem Stand der Technik bekannte rigide oder nicht-rigide Methoden genutzt. Aus jedem der deformierten Referenzbilddatensätze wird anschließend ein Rekonstruktionsbilddatensatz erstellt. Insbesondere wird mittels eines bekannten Rekonstruktionsverfahrens, beispielsweise der sogenannten gefilterten Rückprojektion (filtered back-projection), eine Bilddarstellung (Bild) des Objekts als Rekonstruktionsbilddatensatz erstellt. Alternativ ist der Rekonstruktionsbilddatensatz beispielsweise ein deformiertes Sinogramm.

Beispielsweise ist die Kostenfunktion mittels der Rekonstruktionsbilddatensätze bestimmbar. Hierzu werden diese beispielsweise mit im Trainingsdatensatz hinterlegten und den entsprechenden Referenzbilddatensätzen zugeordneten Trainings-Rekonstruktionsbilddatensätzen verglichen. In analoger Weise zur ersten Variante sind diese Trainings-Rekonstruktionsbilddatensätze beispielsweise mittels eines der eingangs als Stand der Technik genannten Verfahren aus dem entsprechenden Referenzdatensatz ermittelt worden. In analoger Weise werden zur Bestimmung des Ergebnisses der Kostenfunktion hier Differenzen aus den bestimmten Rekonstruktionsdatensätzen und vorbekannten Trainings-Rekonstruktionsdatensätzen herangezogen.

Alternativ wird gemäß einer vorteilhaften Weiterbildung aus den Rekonstruktionsbilddatensätzen eine von der Bewegung des Objekts abhängige Größe ermittelt und zur Bestimmung des Ergebnisses der Kostenfunktion herangezogen. Beispielsweise wird als Ergebnis ein Mittelwert der aus den Rekonstruktionsbilddatensätzen bestimmten Größen verwendet. Hierbei werden also lediglich Referenzdatensätze, aber keine weiteren diesen Referenzdatensätzen zugeordneten Datensätze verwendet. Als Alternative zu dieser Größe wird eine Einschränkung auf positive Werte (positivity constraint) der mittels der Korrekturdatensätze deformierten Referenzdatensätze als Kostenfunktion herangezogen.

In einer geeigneten Weiterbildung wird eine Entropie aus dem entsprechenden Rekonstruktionsbilddatensatz oder eines ausgewählten Teils dessen bestimmt, und als die aus dem Rekonstruktionsbilddatensatz ermittelte Größe herangezogen. Ist der Rekonstruktionsbilddatensatz als ein Bild ausgebildet, so ist als Teil des Bildes zweckmäßigerweise derjenige Bereich des Bildes gewählt, welcher für eine Diagnose relevanter ist (region of interest).

Alternativ oder zusätzlich zur Entropie werden ein Gradient der Entropie und/oder die oben beschriebene Einschränkung auf positive Werte als die aus dem Rekonstruktionsbilddatensatz ermittelte Größe herangezogen.

Gemäß einer besonders geeigneten Ausgestaltung des Verfahrens wird als Maschinenlernverfahren ein (künstliches) neuronales Netz, insbesondere ein convolutional neural network (CNN), herangezogen. Ein neuronales Netz weist dabei eine Eingangsschicht (input layer) auf, in welche Datensätze eingespeist werden, mindesten eine Zwischenschicht (hidden layer) sowie eine Ausgangsschicht (output layer) auf. Dabei umfasst das Netz bzw. die Schichten jeweils eine Anzahl sogenannter Neuronen, welche miteinander verbunden sind. Jede der Verbindungen weist weiter eine Gewichtung auf, welche einen Einfluss eines jeweiligen Sendeneurons auf das entsprechende Zielneuron (Empfangsneuron) bestimmt, wobei die einzelnen Neuronen beispielsweise jeweils als eine mathematische Funktion definiert sind. Die Parameter, welche mittels des Verfahrens bearbeitet werden sind insbesondere die Gewichtungen. Im Zuge der Bearbeitung der Parameter werden insbesondere mehrere Gewichtungen in einem Schritt geändert, wobei die Parameter geeigneter Weise derart geändert werden, dass das ein Fehler der Prognose bzw. der Fehler einer Auswertung sinkt und somit Ergebnis der Kostenfunktion aufgrund der Änderung der Parameter verringert wird. Beispielsweise erfolgt die Änderung der Gewichtungen nach der sog. "gradient descent"- Methode oder nach der sog. "stochastic gradient descent"- Methode.

Ein convolutional neural network (CNN) ist insbesondere für eine Auswertung von Bilddatensätzen geeignet. Beispielsweise alternativ wird ein Neuronales Netz mit vollständig vernetzten (fully connected) Neuronenschichten (FCN) verwendet, wobei jedoch ein convolutional neural network im Vergleich zu einem ein Neuronales Netz mit vollständig vernetzten (fully connected) Neuronenschichten vergleichsweise wenige Parameter aufweist, so dass eine Rechenaufwand des convolutional neural networks vergleichsweise gering ist.

Die vorbekannten Referenzbilddatensätze werden aufgrund des Trainings in das neuronale Netz integriert. Der Vorteil eines neuronalen Netzes besteht insbesondere darin, dass dieses auch ohne eine Unterweisung lernen und arbeiten kann. Die mittels des Trainings vorbekannten Referenzbilddatensätze und die den Bewegungen des Objekts entsprechenden Korrekturdatensätze sind in der Topologie des neuronalen Netzes, also der Struktur des neuronalen Netzes und der Verbindungen der Neuronen untereinander, integriert, so dass bei einer Einspeisung eines auszuwertenden Datensatzes dieser auf vorbekannte Merkmale hin untersucht wird.

In einem Rekonstruktionsverfahren zur Erzeugung eines bewegungskorrigierten Rekonstruktionsbilddatensatzes für einen CT-Bilddatensatz eines bei dessen Aufnahme bewegten Objekts, wird in einem ersten Schritt mittels eines Computertomographiegeräts ein CT-Bilddatensatz eines bewegten Objekts erfasst und bereitgestellt.

In einem darauffolgenden zweiten Schritt wird der CT-Bilddatensatz mittels eines Maschinenlernverfahrens ausgewertet, welches nach einem Verfahren in einem der oben dargelegten Varianten trainiert wurde.

Als Ergebnis der Auswertung wird ein der Bewegung des Objekts entsprechender Korrekturdatensatz bereitgestellt. Mittels diesem und mittels des CT-Bilddatensatzes wird ein bewegungskorrigierter Rekonstruktionsbilddatensatz erzeugt. Hierzu wird der CT-Bilddatensatz gemäß dem Korrekturdatensatz deformiert. Alternativ wird der Korrekturdatensatz als Initialdatensatz für eine iterative Bestimmung mittels eines aus dem Stand der Technik bekannten, beispielsweise eines der eingangs dargelegten, Verfahren verwendet, so dass eine Dauer dieser Verfahren verkürzt ist. Zusammenfassend erfolgt also aufgrund der Verwendung eines, insbesondere als neuronales Netz ausgebildeten, Maschinenlernverfahrens eine Bereitstellung des bewegungskorrigierten Rekonstruktionsdatensatzes vergleichsweise schnell.

Beispielsweise zusätzlich kann das Maschinenlernverfahren mittels dieses CT-Bilddatensatzes und gegebenenfalls mittels des Korrekturdatensatz weiter trainiert werden.

Gemäß einer zweckmäßigen Ausgestaltung weist eine Vorrichtung einen Controller auf, welcher zum Durchführen des Verfahrens in einer der oben dargelegten Varianten und/oder zum Durchführen des Rekonstruktionsverfahrens vorgesehen und ausgebildet ist. Zweckmäßigerweise weist die Vorrichtung des Weiteren ein computerlesbares Medium auf, auf welchem das Maschinenlernverfahren hinterlegt ist.

Nachfolgend wird ein Ausführungsbeispiel der Erfindung anhand einer Zeichnung näher erläutert. Darin zeigen:
- FIG 1: in einem schematischen Ablaufdiagramm ein Verfahren zur Bearbeitung von Parametern eines Maschinenlernverfahrens, in welchem eine Iteration durchgeführt wird, wobei in jedem derer Iterationsschritte ein Ergebnis einer Kostenfunktion ermittelt wird,
- FIG 2: in einem schematischen Ablaufdiagramm eine alternative Ausgestaltung des Verfahrens gemäß FIG 1 mit einer alternativen Bestimmung des Ergebnisses der Kostenfunktion,
- FIG 3: in einem schematischen Ablaufdiagramm ein Rekonstruktionsverfahren zur Erzeugung eines bewegungskorrigierten Rekonstruktionsbilddatensatzes für einen CT-Bilddatensatz,
- FIG 4: ein neuronales Netz zum Durchführen eines Maschinenlernverfahrens, und
- FIG 5: in einer schematischen Frontansicht ein Computertomographiegerät, welches mit einer als Rechner ausgebildeten Vorrichtung verbunden ist, wobei die Vorrichtung einen Controller aufweist.

Einander entsprechende Teile sind in allen Figuren mit den gleichen Bezugszeichen versehen.

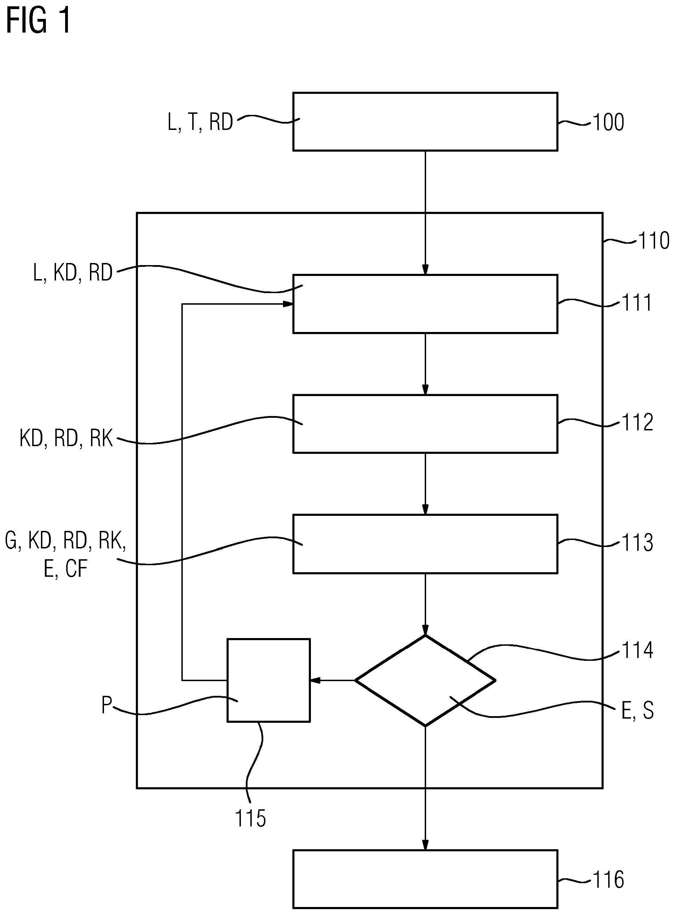

In FIG 1 ist ein Verfahren zur Bearbeitung von Parametern P eines Maschinenlernverfahrens L zur Bereitstellung eines Korrekturdatensatzes KD für eine Bewegungskorrektur eines CT-Bilddatensatzes CB eines bei dessen Aufnahme bewegten Objekts 2 anhand eines Ablaufdiagramms schematisch dargestellt.

In einem ersten Schritt, dem Einleiten 100, wird verfahrensgemäß ein Trainingsdatensatz T mit einer Anzahl an Referenzbilddatensätzen RD bereitgestellt und in das Maschinenlernverfahren L eingeleitet. In einem darauffolgenden zweiten Schritt wird eine Iteration 110 durchgeführt. Die Iteration 110 umfasst dabei mindestens einen Iterationsschritt.

In jedem der Iterationsschritte der Iteration 110 wird mittels des als (künstliches) neuronales Netz ausgebildeten Maschinenlernverfahrens L zunächst ein Korrekturdatensatz KD für jeden der Referenzbilddatensätze RD bestimmt (Schritt 111). Die Referenzbilddatensätze RD werden anschließend mittels der jeweiligen Korrekturdatensätze KD in einem Schritt 112 deformiert und aus den deformierten Referenzbilddatensätzen RD jeweils ein (bewegungs-) korrigierter Rekonstruktionsbilddatensatz RK erstellt.

Ferner wird in einem weiteren Schritt 113 ein Ergebnis E einer Kostenfunktion CF bestimmt. Diese ist dabei von den in diesem Iterationsschritt bestimmten Korrekturdatensätzen KD abhängig. Hierzu wird aus den mittels der Korrekturdatensätze KD und der Referenzbilddatensätze RD erstellten Rekonstruktionsbilddatensätze RK jeweils eine Größe G ermittelt und zur Bestimmung des Ergebnisses E der Kostenfunktion CF herangezogen. Die Größe G ist dabei eine Entropie eines aus dem entsprechenden Rekonstruktionsbilddatensatz RK, beispielsweise die Entropie eines mittels einer gefilterten Rückprojektion (filtered back-projection) aus dem deformierten Referenzbilddatensatz RD ermittelten Bildes. Als Ergebnis E der Kostenfunktion CF wird hier ein Mittelwert der aus den Rekonstruktionsbilddatensätzen RK bestimmten Größen G verwendet.

In einem Vergleichsschritt 114 wird dieses Ergebnis E mit einem, insbesondere vorgegebenen, Schwellenwert S verglichen. Der Schwellenwert S dient dabei zur Beschränkung eines maximal zugelassenen Fehlers oder eine maximal zugelassene Abweichung einer mittels des Maschinenlernverfahrens L gemachten Prognose. Ist das Ergebnis E größer als der Schwellenwert S, die Abweichung bzw. der Fehler also zu groß, so werden Parameter P, hier Gewichtungen des neuronalen Netzes, geändert (Schritt 115). Die Änderung der Gewichtungen P des Neuronalen Netzes L erfolgt nach der sog. "stochastic gradient descent"-Methode. Unterschreitet das Ergebnis E jedoch den Schwellenwert S, so wird die Iteration 110 beendet (Schritt 116). Auf diese Weise werden die Parameter P des Maschinenlernverfahrens L und eine Topologie des neuronalen Netzes bearbeitet.

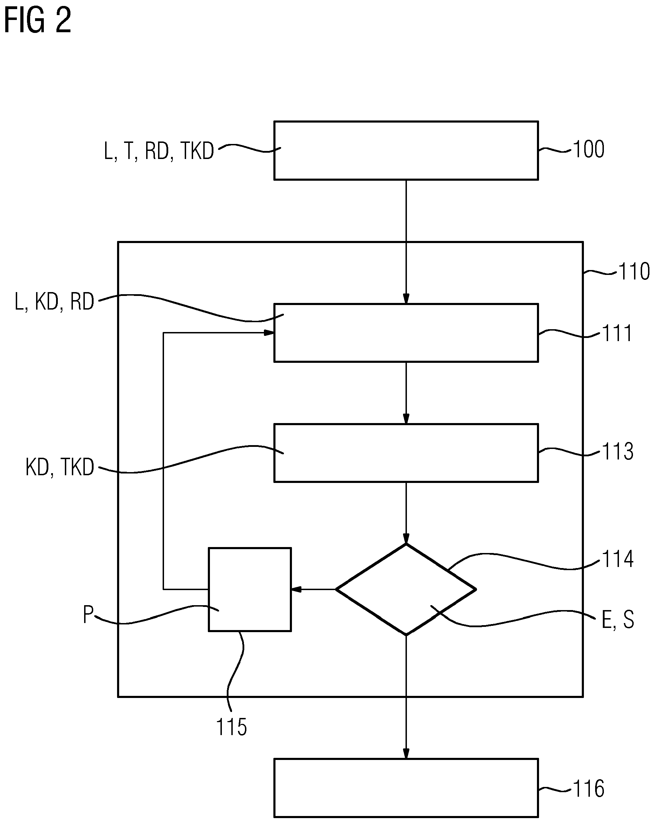

Die FIG 2 zeigt eine alternative Ausgestaltung des Verfahrens. Diese alternative Ausgestaltung unterscheidet sich zu der in FIG 1 gezeigten Variante darin, dass der Trainingsdatensatz TD zusätzlich zu den Referenzbilddatensätzen RD dem entsprechenden Referenzbilddatensatz RD zugeordneten Trainings-Korrekturdatensatz TKD aufweist. In Schritt 113 wird aus den in Schritt 111 bestimmten Korrekturdatensätzen KD und den zugeordneten Trainings-Korrekturdatensatz TKD jeweils eine Differenz aus den entsprechenden Datensätzen KD und TKD bestimmt und zur Bestimmung des Ergebnis E der Kostenfunktion herangezogen. Als Ergebnis E der Kostenfunktion CF wird hier ein Mittelwert der quadrierten Differenzen verwendet.

Schritt 112 findet in der Variante gemäß FIG 2 also nicht statt. Die anderen Schritte des Verfahrens, insbesondere das Beenden der Iteration 110 bei Unterschreitung des Schwellenwertes S durch das Ergebnis E der Kostenfunktion CF (Schritt 116), oder andernfalls der Änderung der Parameter P (Schritt 115) wird analog durchgeführt.

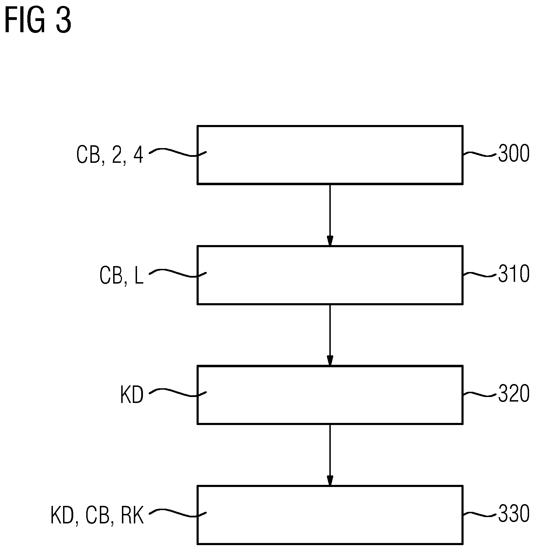

In der FIG 3 ist ein Rekonstruktionsverfahren zur Erzeugung eines bewegungskorrigierten Rekonstruktionsbilddatensatzes RK für einen CT-Bilddatensatz CB eines bei dessen Aufnahme bewegten Objekts 2 dargestellt. Hierbei wird in einem ersten Schritt 300 mittels eines Computertomographiegeräts 4 der CT-Bilddatensatz CB des bewegten Objekts 2 erfasst und bereitgestellt.

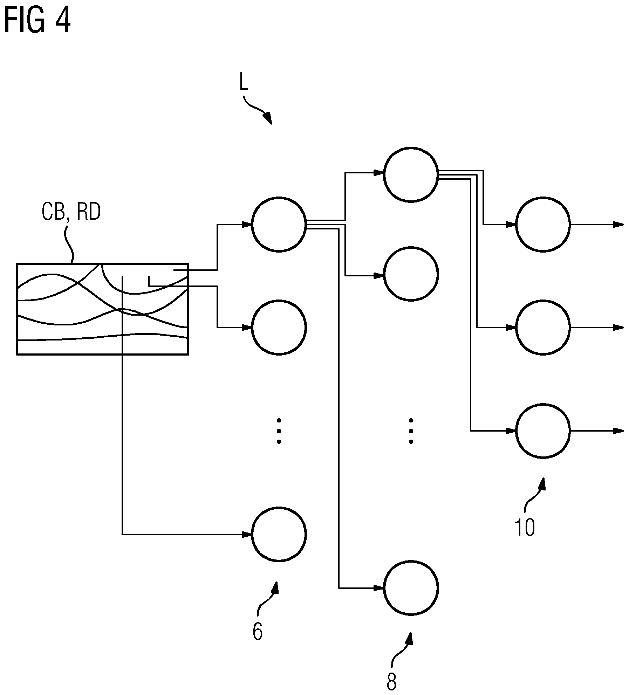

In einem darauffolgenden zweiten Schritt 310 wird der CT-Bilddatensatz CB mittels des Maschinenlernverfahrens L ausgewertet (FIG 4). Dieses ist gemäß einem der Verfahren der FIG 1 oder der FIG 2 trainiert worden.

Als Ergebnis der Auswertung wird ein der Bewegung des Objekts 2 entsprechender Korrekturdatensatz KD bereitgestellt (Schritt 320). Mittels diesem und mittels des CT-Bilddatensatzes CB wird in einem Schritt 330 ein bewegungskorrigierter Rekonstruktionsbilddatensatz RK erzeugt. Hierzu wird der CT-Bilddatensatz CB gemäß des Korrekturdatensatzes KD deformiert, d.h. der CT-Bilddatensatz CB wird entsprechend des bestimmten Korrekturdatensatzes KD geändert. Auf diese Weise wird die Bewegung des Objekts 2 bei der Aufnahme des CT-Bilddatensatzes CB korrigiert und Bewegungsartefakte in dem als Bild ausgebildeten Rekonstruktionsbilddatensatz RK vermieden. Aufgrund dessen ist ein Fehler bei einer Interpretation bzw. bei einer Diagnose anhand des nun bewegungskorrigierten Bildes vermieden.

In FIG 4 ist schematisch und stark vereinfacht ein neuronales Netz L dargestellt, welches in den Verfahren der FIG 1 bis FIG 3 verwendet wird. Das neuronale Netz L weist eine (input layer) Eingangsschicht 6 auf, in welche Datensätze eingespeist werden, mindesten eine (hidden layer) Zwischenschicht 8 sowie eine (output layer) Ausgangsschicht 10 auf. Die Schichten 6, 8 und 10 wiederum umfassen jeweils eine Anzahl an von nicht näher bezeichneten Neuronen, welche miteinander verbunden sind und jeweils als eine mathematische Funktion definiert sind. Zum Zwecke einer verbesserten Übersicht sind in der FIG 10 dabei beispielhaft lediglich neun Verbindungen zu Neuronen gezeigt, jedoch sind in dieser Ausführung die Neuronen der Eingangsschicht 6 jeweils mit allen Neuronen der Zwischenschicht 8 und die Neuronen der Zwischenschicht 8 jeweils mit allen Neuronen der Ausgangsschicht 10 verbunden. Jede dieser Verbindungen weist dabei eine Gewichtung P auf, welche einen Einfluss eines jeweiligen Sendeneurons auf das entsprechende Zielneuron bestimmt.

Aus dem CT-Bilddatensatz CB bzw. während der Trainingsphase aus den Referenzbilddatensätzen RD wird die Eingangsschicht 6 mit einer Anzahl an Neuronen bestimmt. Hierbei entspricht beispielsweise jeder Pixel oder jeweils eine Matrix an Pixeln des als Sinogramm eingespeisten Bilddatensatzes einem Neuron der Eingangsschicht 6.

Die Merkmale einer Bewegung des Objekts 2 in den Referenzbilddaten RD sind in mathematischen Funktionen und/oder Gewichtungen der Neuronen des neuronalen Netzes integriert. Mit anderen Worten sind die Bewegungsmerkmale der Referenzbilddaten RD dem neuronalen Netz antrainiert und in dessen Topologie (FIG 4) integriert. Auf diese Weise extrahiert und erkennt die mindestens eine Zwischenschicht (verdeckte Schicht) 8 charakteristische Bewegungsmerkmale des zugeführten Datensatzes. Bei einer Auswertung eines eingespeisten Datensatzes wird somit eine Bewegung erkannt und der entsprechende Korrekturdatensatz KD Für eine Korrektur dieser Bewegungsmerkmale als Ergebnis E an den (Ausgangs-) Neuronen der Ausgangsschicht 10 ausgebgeben.

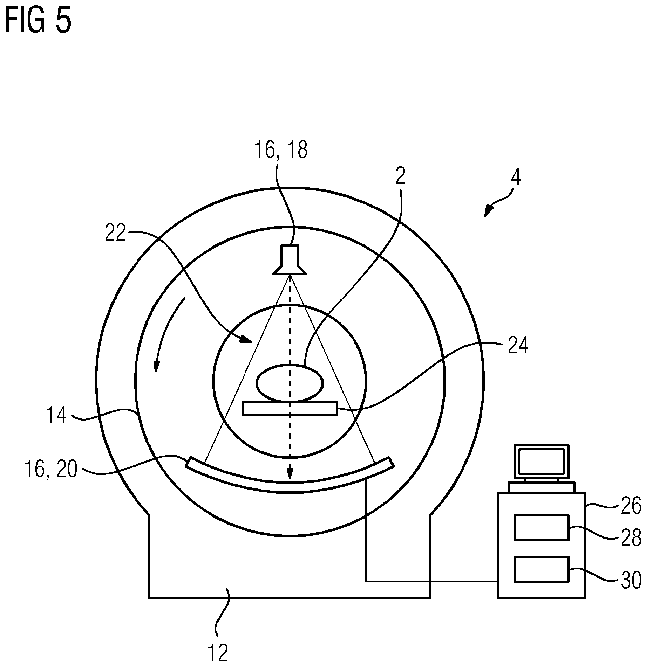

IN FIG 5 ist ein Computertomographiegerät 4 gezeigt. Dieses weist einen Halterahmen 12 für einen ringförmigen Drehkranz 14 auf, welcher auch als Gantry bezeichnet wird. Dabei ist der Drehkranz 14 drehbar am Halterahmen gelagert 12. Der Drehkranz 14 trägt ein Röntgensystem 16 mit einer Röntgenquelle 18 zur Erzeugung von Röntgenstrahlen und mit einem in Gegenüberstellung zu dieser angeordneten (Röntgen-) Detektor 20 für die Röntgenstrahlen. In den mittels des ringförmigen Drehkranzes 14 gebildeten, tunnelförmigen Aufnahmebereich 22 ist eine Patientenliege 24 ein- oder verfahrbar. Für eine Aufnahme eines CT-Bilddatensatzes wird das zu untersuchendes Objekt 2, insbesondere ein Patient, auf der Patientenliege 22 platziert und in den Aufnahmebereich 22 verfahren. Bei einer Drehung des Drehkranz 14 das sich auf der Patientenliege 24 befindende Objekt 2 kreisförmig vom Röntgensystem 10 umfahren. Folglich wird ein CT-Bilddatensatz CB des Objekts 2 aus unterschiedlichen (Raum-, Aufnahme-) Winkeln aufgenommen, wobei der CT-Bilddatensatz CB einen Intensitätsverlauf der einfallenden Röntgenstrahlung entsprechend der unterschiedlichen (Aufnahme-) Winkel repräsentiert.

Zur Erfassung des mittels des Detektors 20 aufgenommenen (erfassten) CT-Bilddatensatzes CB ist dieser mit einem als Rekonstruktionsrechner ausgebildeten Vorrichtung 26 signalübertragungstechnisch verbunden. Dieser weist einen Controller 28 zur Durchführung des in FIG 1 bzw. FIG 2 gezeigten Verfahrens und des in FIG 3 gezeigten Rekonstruktionsverfahren, sowie ein als Festplatte ausgebildetes computerlesbares Medium 30 auf, auf welcher das als neuronales Netz ausgebildete Maschinenlernverfahren L der FIG 1 bis FIG 4 hinterlegt ist.

Die Erfindung ist nicht auf die vorstehend beschriebenen Ausführungsbeispiele beschränkt. Vielmehr können auch andere Varianten der Erfindung vom Fachmann hieraus abgeleitet werden, ohne den Gegenstand der Erfindung zu verlassen. Insbesondere sind ferner alle im Zusammenhang mit den Ausführungsbeispielen beschriebenen Einzelmerkmale auch auf andere Weise miteinander kombinierbar, ohne den Gegenstand der Erfindung zu verlassen.

## Patentansprüche

1. Computerimplementiertes Verfahren zur Bearbeitung von Parametern (P) eines Maschinenlernverfahrens (L) zur Bereitstellung eines Korrekturdatensatzes (KD) für eine Bewegungskorrektur eines CT-Bilddatensatzes (CB) eines bei dessen Aufnahme bewegten Objekts (2),
- wobei ein Trainingsdatensatz (T) mit einer Anzahl an Referenzbilddatensätzen (RD) zum Einleiten in das Maschinenlernverfahren (L) bereitgestellt wird (100),
- wobei eine Iteration (110) durchgeführt wird, wobei in jedem derer Iterationsschritte für jeden der Referenzbilddatensätze (RD) ein Korrekturdatensatz (KD) mittels des Maschinenlernverfahrens (L) bestimmt (111) und ein Ergebnis (E) einer von den in diesem Iterationsschritt bestimmten Korrekturdatensätzen (KD) abhängigen Kostenfunktion (CF) ermittelt wird (113), und
- wobei in Abhängigkeit (114) des Ergebnisses (E) der Kostenfunktion (CF) die Iteration (110) beendet (116) oder ein Parameter (P) des Maschinenlernverfahrens (L) geändert wird (115),
wobei zur Bestimmung des Ergebnisses (E) der Kostenfunktion (CF) Differenzen aus den bestimmten Korrekturdatensätzen (KD) und vorbekannten Trainings-Korrekturdatensätzen (TKD) herangezogen werden, wobei die Trainings-Korrekturdatensätze (TKD) im Trainingsdatensatz (T) hinterlegt und den entsprechenden Referenzbilddatensätzen (RD) zugeordnet sind, oder
wobei zur Bestimmung des Ergebnisses (E) der Kostenfunktion (CF) jeder der Referenzbilddatensätze (RD) entsprechend des zugeordneten Korrekturdatensatzes (KD) deformiert (112) und aus dem deformierten Referenzbilddatensatz (RD) ein Rekonstruktionsbilddatensatz (RK) erstellt wird.

2. Verfahren nach Anspruch 1, wobei zur Bestimmung des Ergebnisses (E) der Kostenfunktion (CF) jeder der Referenzbilddatensätze (RD) entsprechend des zugeordneten Korrekturdatensatzes (KD) deformiert und aus dem deformierten Referenzbilddatensatz (RD) ein Rekonstruktionsbilddatensatz (RK) erstellt wird, **dadurch gekennzeichnet, dass** aus den Rekonstruktionsbilddatensätzen (RK) eine von der Bewegung des Objekts (2) abhängige Größe (G) ermittelt und zur Bestimmung des Ergebnisses (E) der Kostenfunktion (CF) herangezogen wird.

3. Verfahren nach Anspruch 2, wobei zur Bestimmung des Ergebnisses (E) der Kostenfunktion (CF) jeder der Referenzbilddatensätze (RD) entsprechend des zugeordneten Korrekturdatensatzes (KD) deformiert und aus dem deformierten Referenzbilddatensatz (RD) ein Rekonstruktionsbilddatensatz (RK) erstellt wird, **dadurch gekennzeichnet, dass** eine Entropie aus dem Rekonstruktionsbilddatensatz (RK) oder eines ausgewählten Teils dessen bestimmt und als die aus dem Rekonstruktionsbilddatensatz (RK) ermittelte Größe (G) herangezogen wird.

4. Verfahren nach einem der Ansprüche 1 bis 3,
**dadurch gekennzeichnet, dass** als Maschinenlernverfahren (L) ein neuronales Netz, insbesondere ein convolutional neural network, herangezogen wird.

5. Computerimplementiertes Rekonstruktionsverfahren zur Erzeugung eines bewegungskorrigierten Rekonstruktionsbilddatensatzes (RK) für einen CT-Bilddatensatz (CB) eines bei dessen Aufnahme bewegten Objekts (2),
- wobei mittels eines Computertomographiegeräts (4) der CT-Bilddatensatz (CB) des bewegten Objekts (2) erfasst und bereitgestellt wird,
- wobei der CT-Bilddatensatz (CB) mittels eines nach einem der Ansprüche 1 bis 4 trainierten Maschinenlernverfahrens (L) ausgewertet wird,
- wobei ein der Bewegung des Objekts (2) entsprechender Korrekturdatensatz (KD) als Ergebnis der Auswertung bereitgestellt wird, und
- wobei mittels des Korrekturdatensatzes (KD) und des CT-Bilddatensatzes (CB) ein bewegungskorrigierter Rekonstruktionsbilddatensatz (RK) erzeugt wird.

6. Vorrichtung (26) mit einem Controller (28) zum Durchführen des Verfahrens nach einem der Ansprüche 1 bis 4 und/oder zum Durchführen des Rekonstruktionsverfahrens nach Anspruch 5.

## Claims

1. Computer-implemented method for processing parameters (P) of a machine learning method (L) for providing a correction data record (KD) for a movement correction of a CT image data record (CB) of an object (2) moving during its capture,
- wherein a training data record (T) with a number of reference image data records (RD) is provided (100) for introduction into the machine learning method (L),
- wherein an iteration (110) is carried out, wherein a correction data record (KD) is determined (111) by means of the machine learning method (L) in each of their iteration steps for each of the reference image data records (RD) and a result (E) of a cost function (CF) dependent on the correction data records (KD) determined in this iteration step is determined (113), and
- wherein the iteration (110) is terminated (116) or a parameter (P) of the machine learning method (L) is modified (115) as a function (114) of the result (E) of the cost function (CF),
wherein in order to determine the result (E) of the cost function (CF), differences from the determined correction data records (KD) and previously known training correction data records (TKD) are used, wherein the training correction data records (TKD) are stored in the training data record (T) and assigned to the corresponding reference image data records (RD) or
wherein in order to determine the result (E) of the cost function (CF), each of the reference image data records (RD) is deformed (112) according to the assigned correction data record (KD) and a reconstruction image data record (RK) is created from the deformed reference image data record (RD).

2. Method according to claim 1, wherein in order to determine the result (E) of the cost function (CF), each of the reference image data records (RD) is deformed according to the assigned correction data record (KD) and a reconstruction image data record (RK) is created from the deformed reference image data record (RD), **characterised in that** a variable (G) dependent on the movement of the object (2) is determined from the reconstruction image data records (RK) and used to determine the result (E) of the cost function (CF).

3. Method according to claim 2, wherein in order to determine the result (E) of the cost function (CF), each of the reference image data records (RD) is deformed according to the assigned correction data record (KD) and a reconstruction image data record (RK) is created from the deformed reference image data record (RD), **characterised in that** an entropy is determined from the reconstruction image data record (RK) or a selected part thereof and used as the variable (G) determined from the reconstruction image data record (RK).

4. Method according to one of claims 1 to 3, **characterised in that** a neural network, in particular a convolutional neural network, is used as a machine learning method (L).

5. Computer-implemented reconstruction method for generating a movement-corrected reconstruction image data record (RK) for a CT image data record (CB) of an object (2) moving during its capture,
- wherein the CT image data record (CB) of the moving object (2) is captured and provided by means of a computed tomography device (4),
- wherein the CT image data record (CB) is evaluated by means of a machine learning method (L) trained according to one of claims 1 to 4,
- wherein a correction data record (KD) corresponding to the movement of the object (2) is provided as a result of the evaluation, and
- wherein a movement-corrected reconstruction image data record (RK) is generated by means of the correction data record (KD) and the CT image data record (CB).

6. Device (26) with a controller (28) for implementing the method according to one of claims 1 to 4, and/or for carrying out the reconstruction method according to claim 5.

## Revendications

1. Procédé mis en oeuvre par ordinateur de traitement de paramètres (P) d'un procédé (L) d'enseignement par machine pour disposer d'un ensemble (KD) de données de correction pour une correction de déplacement d'un ensemble (CB) de données d'image CT d'un objet (2) déplacé lors de son enregistrement,
- dans lequel on se procure (100) un ensemble (T) de données d'apprentissage ayant un nombre d'ensembles (RD) de données d'image de référence à introduire dans le procédé (L) d'enseignement par machine,
- dans lequel on exécute une itération (110), dans lequel, dans chacun des stades d'itération, on détermine (111), pour chacun des ensembles (RD) de données d'image de référence, un ensemble (KD) de données de correction au moyen du procédé (L) d'enseignement par machine, et on détermine (113) un résultat (E) d'une fonction (CF) de coût, en fonction des ensembles (KD) de données de correction déterminés dans ce stade d'itération, et
- dans lequel, en fonction (114) du résultat (E) de la fonction (CF) de coût, on met fin (116) à l'itération (110) ou on modifie (115) un paramètre (P) du procédé (L) d'enseignement par machine,
dans lequel, pour la détermination du résultat (E) de la fonction (CF) de coût, on tire parti de différences entre les ensembles (KD) de données de correction déterminés et des ensembles (TKD) de données de correction d'apprentissage connues auparavant, dans lequel les ensembles (TKD) de données de correction d'apprentissage sont mis en mémoire dans l'ensemble (T) de données d'apprentissage et sont associées aux ensembles (RD) de données d'image de référence correspondants, ou
dans lequel, pour la détermination du résultat (E) de la fonction (CF) de coût, on déforme (112) chacun des ensembles (RD) de données d'image de référence, de manière correspondante avec l'ensemble (KD) de données de correction associé et on établit un ensemble (RK) de données d'image de reconstruction à partir de l'ensemble (RD) de données d'image de référence déformé.

2. Procédé suivant la revendication 1, dans lequel, pour la détermination du résultat (E) de la fonction (CF) de coût, on déforme chacun des ensembles (RD) de données d'image de référence de manière correspondante avec l'ensemble (KD) de données de correction associé et on établit un ensemble (RK) de données d'image de reconstruction à partir de l'ensemble (RD) de données d'image de référence déformé, **caractérisé en ce que**, à partir des ensembles (RK) de données d'image de reconstruction, on détermine une grandeur (G), qui dépend du déplacement de l'objet (2) et on en tire parti pour la détermination du résultat (E) de la fonction (CF) de coût.

3. Procédé suivant la revendication 2, dans lequel, pour la détermination du résultat (E) de la fonction (CF) de coût, on déforme chacun des ensembles (RD) de données d'image de référence de manière correspondante avec l'ensemble (KD) de données de correction associé et, à partir de l'ensemble (RD) de données d'image de référence déformé, on établit un ensemble (RK) de données d'image de reconstruction, **caractérisé en ce que** l'on détermine une entropie à partir de l'ensemble (RK) de données d'image de reconstruction ou d'une partie sélectionnée de celui-ci et on en tire parti comme grandeur (G) déterminée à partir de l'ensemble (RK) de données d'image de reconstruction.

4. Procédé suivant l'une des revendications 1 à 3, **caractérisé en ce que** l'on tire parti, comme procédé (L) d'enseignement par machine, d'un réseau neuronal, en particulier d'un réseau neuronal de convolution.

5. Procédé de reconstruction mis en oeuvre par ordinateur pour la production d'un ensemble (RK) de données d'image de reconstruction corrigé en déplacement pour un ensemble (CB) de données d'image CT d'un objet (2) déplacé lors de son enregistrement,
- dans lequel, au moyen d'un appareil (4) de tomodensitométrie assistée par ordinateur, on saisit et on se procure l'ensemble (CB) de données d'image CT de l'objet (2) déplacé,
- dans lequel on exploite l'ensemble (CB) de données d'image CT au moyen d'un procédé (L) d'enseignement par machine ayant subi un apprentissage suivant l'une des revendications 1 à 4,
- dans lequel on se procure, comme résultat de l'exploitation, un ensemble (KD) de données de correction correspondant au déplacement de l'objet (2), et
- dans lequel, au moyen de l'ensemble (KD) de données de correction et de l'ensemble (CB) de données d'image CT, on produit un ensemble (RK) de données d'image de reconstruction corrigé en déplacement.

6. Installation (26) comprenant une unité (28) de commande pour effectuer le procédé suivant l'une des revendications 1 à 4 et/ou pour effectuer le procédé de reconstruction suivant la revendication 5.
